Europäisches Patentamt

European Patent Office

Office européen des brevets

(19)

(11) Publication number: **0 161 384**
A2

# EUROPEAN PATENT APPLICATION

(21) Application number: **85100221.2**

(22) Date of filing: **11.01.85**

(51) Int. Cl.⁴: **C 12 P 21/00,** C 12 N 5/00,
A 61 K 37/02

(30) Priority: **17.05.84 JP 99370/84**

(43) Date of publication of application: **21.11.85**
**Bulletin 85/47**

(84) Designated Contracting States: **AT BE CH DE FR GB IT
LI LU NL SE**

(71) Applicant: **YAMANOUCHI PHARMACEUTICAL CO. LTD.,
No. 5-1 Nihonbashi-Honcho, 2-chome Chuo-ku, Tokyo
(JP)**

(72) Inventor: **Obara, Tadashi, No. 1-1-15-1007, Kamiochiai
Shinjuku-ku, Tokyo (JP)**
Inventor: **Ezoe, Hisanori, No. 1-43-8, Uehara Shibuya-ku,
Tokyo (JP)**
Inventor: **Haranaka, Katsuyuki, No. 2-31-10, Hiyoshi-cho,
Kokubunji-shi Tokyo (JP)**
Inventor: **Satomi, Nobuko, No. 1-1-11-1103, Ohashi
Meguro-ku, Tokyo (JP)**

(74) Representative: **Wuesthoff, Franz, Dr.-Ing. et al,
Patentanwälte Wuesthoff -v. Pechmann-Behrens-Goetz
Schweigerstrasse 2, D-8000 München 90 (DE)**

(54) **Human endogenous cancer regulatory factors, method of preparing the same, and pharmaceutical compositions containing the same.**

(57)     Human endogenous cancer regulatory factors useful as anti-tumor agent having a molecular weight from 50,000 to 72,000 and an isoelectric point in the pH range from 6.0 to 8.5, and pharmaceutical compositions containing the same, are disclosed. The human endogenous cancer regulatory factors of the present invention are obtained by growing human monocytic cells or cloned strains therefrom in a tissue culture medium to form said cancer regulatory factors, followed by isolation from the culture medium and purification.

Patentanwälte
Wuesthoff - v. Pechmann
Behrens - Goetz
Schweigerstraße 2
8000 München 90

TITLE OF THE INVENTION

Human endogenous cancer regulatory factors, method of preparing the same, and pharmaceutical compositions containing the same

The inventors formerly discovered that a novel biologically active substance can be produced by growing human monocytic leukemia, cells in a tissue culture medium, followed by addition of 12-O-tetradecanoyl-phorbol-13-acetate ( TPA ) and of a lipopolysaccharide in that order (Japanese Patent Application No.128893, 1983 ). This is a biologically active substance having powerful activity, both in vitro and in vivo, against many kinds of tumors including human tumor cells and significantly differing from the known human tumor necrosis factor ( TNF ) in physicochemical properties such as molecular weight, isoelectric point and stability. This material, derived from human cells, was considered to be one of the substances that are produced in very small amounts in the body of a patient who has spontaneously recovered from a cancer because of its ability to cause tumor necrosis. We therefore named this material a human endogenous cancer regulatory factor ( Krebs Statika; KBS).

Its relative molecular weight was 82,000 ± 10,000 and isoelectric point was pH 6.5 ± 0.5.

Further studies have led us to find that

-2-

0161384

biologically active materials differing from KBS in molecular weight and other physicochemical properties can be obtained when a cloned cell line (for example clone YKBS-4B3) derived from YKBS-7-15, the human monocytic leukemia cells used for the production of KBS, is grown in a tissue culture, followed by addition of TPA and of a lipopolysaccharide in that order.  Like KBS, this new biologically active material, derived from human cells, has anti-tumor activity and is considered to be one of human endogenous cancer regulatory factors produced in very small amounts during spontaneous recover   from a cancer.  The human endogenous cancer regulatory factor of the present invention consists  of one, two or three substances having the same relative molecular weight of about 60,000 but differing in isoelectric point and other properties.  We therefore named the substance having an isoelectric point at about pH 6.5 human endogenous cancer regulatory factor-α ( Krebs statika-α, hereinafter abbreviated as "KBS-α" ), named the substance having an isoelectric point of about pH 7.0 human endogenous cancer regulatory factor-β ( Krebs Statika-β; hereinafter abbreviated as "KBS-β" ), and named the substance having an isoelectric point of about pH 8.0 human endogenous cancer regulatory factor-γ ( Krebs Statika-γ; hereinafter abbreviated as "KBS-γ" ).

-3-

The human endogenous cancer regulatory factors of the present invention, KBS-α, KBS-β and KBS-γ, are new proteins distinct from KBS which the inventors formerly isolated, because their relative molecular weight is approximately 60,000 ( more precisely, in the range from 50,000 to 72,000 ) in contrast to 82,000 ± 10,000 for the latter. KBS-α, KBS-β and KBS-γ are considered to be proteins slightly differing from one another in amino acid and sugar compositions, judging from the difference in isoelectric point. These three factors have high ability to cause tumor necrosis in nude mice to which human cancer cells have been transplanted, and show, like KBS, powerful anti-tumor activity against many kinds of tumor cells.

In addition, there is valid reason to consider that KBS-α, KBS-β and KBS-γ, which are biological substances derived from human cells like KBS, will not be recognized as heterologous to human and therefore will cause little, if any, antigen-antibody reaction.

## BRIEF DESCRIPTION OF THE DRAWINGS

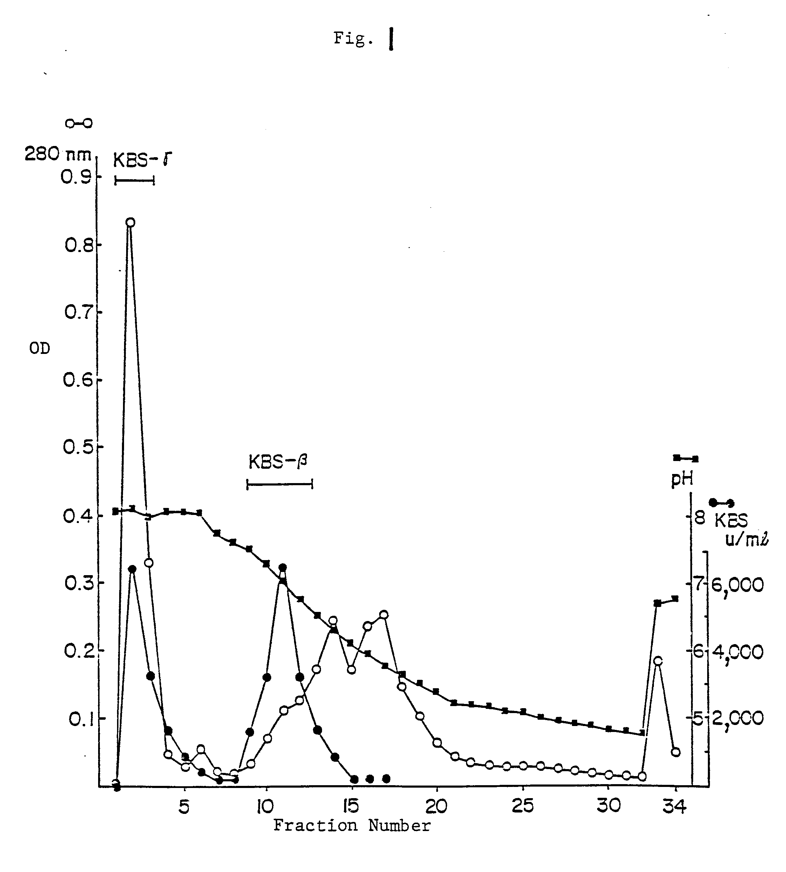

Figs. 1, 2, 3 and 6 show elution profiles of chromatofocusing for KBS-α, KBS-β and KBS-γ on FPLC/Mono P column performed in Example 1, in which the open circles (o—o) illustrate absorbance of protein at 280nm, the closed circles (●—●) KBS activity,

-4-

the closed squares ( ■——■ ) pH value, and the fraction number is plotted on the abscissa.

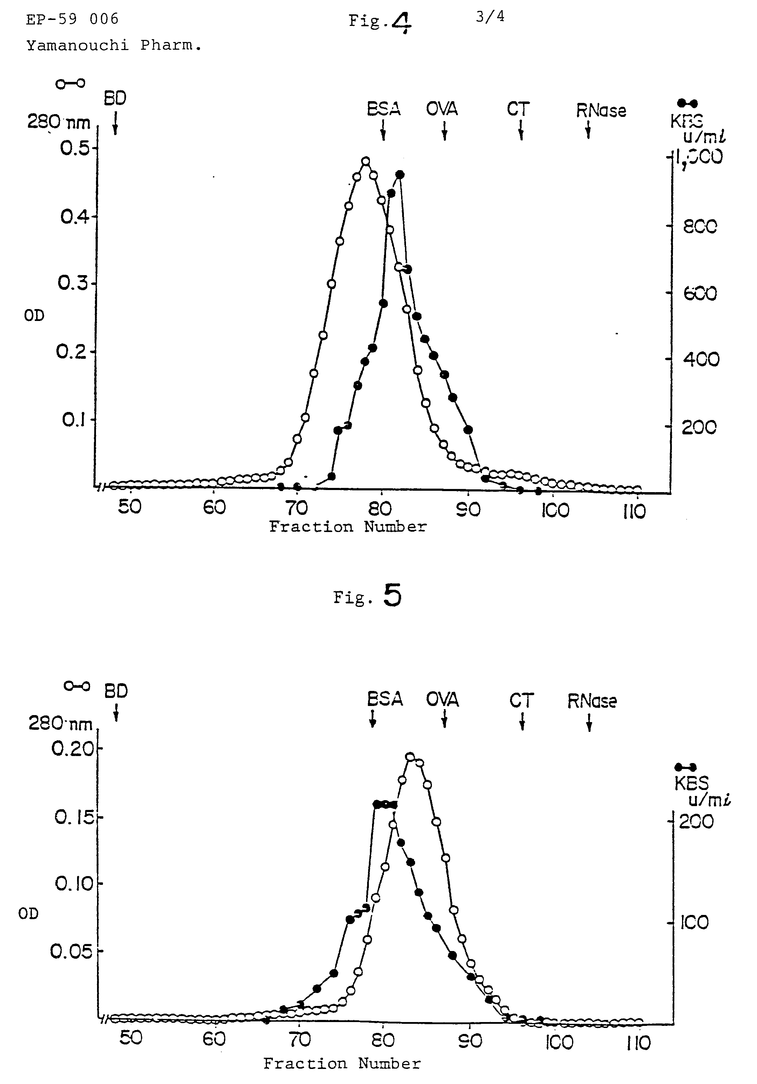

Figs. 4, 5 and 7 show elution profiles of column chromatography for KBS-⋋, KBS-β and KBS-ɣ on Sephadex G-200 conducted in Example 1, in which the arrows ( → ) indicate the elution positions for reference substances [ BD: Blue Dextran; BSA: bovin serum albumin ( M.W.: 67,000); OVA: ovalbumin ( M.W.: 43,000 ); CY: chymotrypsin (M.W.: 25,000 ); RNase: ribonuclease ( M.W.: 13,700 ) ], the open circles ( o——o ) indicate absorbance of protein at 280nm, the closed circles ( ●——● ) indicate KBS activity, and the fraction number is plotted on the abscissa.

KBS-⋋, KBS-β and KBS-ɣ of the present invention are produced by growing human, normal or leukemic cells in a tissue culture medium and recovering these factors thus formed from the culture medium.

The normal or leukemic monocytic cells as used in the present invention include normal monocyte and macrophage cells, as well as their leukemic cells. Those which can be easily proliferated in a tissue culture medium are more preferable.

Particularly preferred are leukemic cells of monocyte/macrophage series. Typical examples include HL-60; YKBS-7-15, YKBS-7-16 and YKBS-7-17 which are monocytic leukemia cells selected from clinically established peripheral leukocytes of monocytic leukemia origin; and cloned strains therefrom, such as clones 2A2, 2A6, 2C6, 3A1, 3B3, 3B4, 3C1, 3D5, 4B3 and others cloned from YKBS-7-15.

The method of cloning conducted is a limiting dilution and will be described as follows, as example, as to cloned cell lines from the already isolated and established cell line YKBS-7-15 (Japanese Patent Application No. 239355,1983).

Cultured cells of YKBS-7-15 ( monocytic leukemic cells ), derived from human leukemic peripheral leukocytes, were placed on a 96-well microplate, previously seeded with $1 \times 10^6$ mouse thymocytes as feeder, at a concentration of 1 to 2 cell/well ( limiting dilution method ), and grown at 37°C under 5% $CO_2$. When colonies are visible, subculture was carried out by transferring in turn to a 24-well microplate, and clones highly capable of KBS production, such as 2A2, 2A6, 2C6, 3A1, 3B3, 3B4, 3C1, 3D5 and 4B3 were selected.

The established cell lines, YKBS-7-15, YKBS-7-16 and YKBS-7-17, are monocytic leukemia cell lines which the inventors and the Institute of Medical Research Center of Tokyo University have succeeded in isolating and establishing by culturing the buffy coat leukocytes from the peripheral blood of a patient suffering acute monocytic leukemia by a usual method ( ammonium chloride treatment for removal of erythrocytes ). Of these, YKBS-7-15, and YKBS-4B3 which is an isolated

0161384

clone therefrom, have been deposited with the C.N.C.M. of Pasteur Institute, France (I-258 and I-319, respectively).

Other examples of cell lines of monocyte/macrophage series include Mono-1 and Mono-1-207 [ Virchow Arch. A. Pathol. Anat. Histol 371, 15 ( 1976 ) and 379, 269 ( 1978)].

In addition, cells capable of differentiation into monocytic cells may of course be used in the present invention. These cells eventually exhibit the properties of monocytic leukemia, and malignant myeloid cells are one example.

The cells listed above are subjected to tissue culture usually in a growth medium containing fetal calf serum ( FCS ). For example YKBS-4B3, a clone of YKBS-7-15 used in Example 1, can be successfully grown in RPMI mdeium ( Gibco Co. ) containing 10% FCS, 15mM HEPES ( Wako Junyaku Co. ), and 50 mg/l kanamycin and 25 mg/l streptomycin. In this formulation, penicillin, gentamycin, sisomicin and other antibiotics may also be used in place of kanamycin and streptomycin. It is also possible to perform the tissue culture in serum-free growth media, such as HB101$^{TM}$ ( Hana Biologics. Inc. ) and ISCOVE'S media (Flow Laboratories ). In addition, the cells may also be proliferated intraperitoneally or subcutaneously in non-human, warm-blooded animals like nude mice and juvenile hamsters. Tissue culture medium as used in the present application includes such *in vivo* growth media.

Examples of the first stimulant used in the present invention include conditioned media with lymphocytes or lymphoblasts, chemical substances or natural extracts that induce leukocytes by differentiation, and mixtures thereof. Another example is the supernatant which is obtained by culturing sensitized cells in animals administered with a certain chemical susbstance such as Tricothecene mycotoxin. One example of the chemical substances or natural extracts that induce leukocytes . by differentiation is phytohemagglutinin ( PHA ), but macrophage activating substances, such as muramyl dipeptide ( MDP ), 12-O-tetradecanoylphorbol-13-acetate ( TPA ), 12,13-phorbol butyrate, dimethylsulfoxide ( DMSO ) and mezerein are particularly preferred.

In addition, substances capable of activating the reticuloendothelial system may also be used as the first stimulant in the present invention. Common examples include Gram-positive bacteria, fungus-produced materials, protozoans and yeasts, which are used in the form of live cells, dead cells ( after heat or formalin treatment, for example ) or cell extracts. Illustrative Gram-positive bacteria may be mentioned Propioni bacteria, such as Pro-pionibacterium acnes ( Corynebacterium parvum ) and Pro-

pionibacterium granulocum ( Corynebacterium granulocum );

Mycobacteria, such as Bacillus Calmette-Guerin ( B.C.G.) and Mycobacterium smegmatis; and Nocardia, such as Nocardia erythropolis and Nocardia gardnerl. Illustrative fungus-produced materials are toxins produced by Fusarium. Typical protozoans are Plasmodium and Toxoplasma. A commonly used yeast is Zymosan extracted from Saccharomyces cerevisiae or the like. Certain synthetic polymers such as pyran copolymers may also be employed as the first stimulant.

The second stimulant used in the present invention. is an endotoxin produced from Gram-positive or Gram-negative bacteria. Typical examples include lipopolysaccharides derived from E. coli, Pseudomonas aeruginosa and typhoid bacillus. Lipopolysaccharides from Gram-positive bacteria can also be used with good results.

KBS-$\alpha$, KBS-$\beta$ and KBS-$\gamma$ of the present invention can be produced by growing normal human cells or human monocytic leukemia cells in a growth medium or a common tissue culture medium containing serum and other nutrients, and adding the first stimulant before, during or after cultivation, followed by addition of the second stimulant. Although both the two stimulants ( first and second ) are commonly used for induction, these factors could be produced only by the action of either one of the two stimulants, or even in the absence of any stimulant. Most commonly, however, the cells are fully proliferated in a usual tissue culture medium, and the first stimulant ( for example, 0.1 to 100ng/ml of TPA ) is then added to induce the first stimulation, followed by addition of the second stimulant ( for example, 0.1 to 10μg/ml of lipopolysaccharide derived from E. coli ) to induce the second stimulation, thus yielding, after further cultivation for a certain period, say, 8 to 24 hours, KBS-α, KBS-β and KBS-γ in the supernatant.

The three human endogenous cancer regulatory factors thus produced can be recovered from the culture medium and purified by known techniques, such as dialysis, salting-out, ultrafiltration, centrifugation, concentration and freeze-drying. If further purification is necesary, these techniques may be combined with such additional operations as adsorption on ion exchangers followed by elution therefrom, gel filtraton, affinity chromatography using Concanavalin A ( Con A ), a suitable antibody or the like supported on Sepharose, isoelectric fractionation, high-performance liquid chromatography, chromatofocusing by means of high-performance liquid chromatography for proteins, ion exchanging ( for example, FPLC/Mono-P and Mono-Q columns; Pharmacia AB), and slab or other types of electrophoresis on polyacrylamide gel. Usually, the supernatant containing KBS-α, KBS-β and KBS-γ is separated from the culture medium by centrifugation and then treated with an anion exchanger, followed by further purification by other techniques. Suitable anion exchangers include DEAE-Sephadex A-50, DEAE-Sepharose CL-6B, DEAE-Sephacel and QAE-Sephadex ( products of Pharmacia AB ), AIECDE 52 ( Wattman Co. ), Servacel AE ( Serva Co. ) and Cellex QAE ( Bio-rad Laboratories).

When a supernatant containing KBS-$\alpha$, KBS-$\beta$ and KBS-$\gamma$, after the salt concentration being diluted to adequately lower, is allowed to flow batchwise through a Buchner funnel loaded with an anion exchanger, such as DEAE-Sephadex, KBS-$\alpha$ will be adsorbed on the ion exchanger, giving a fraction containing KBS-$\beta$ and KBS-$\gamma$ left unadsorbed. KBS-$\beta$ and KBS-$\gamma$ can be isolated in pure form by subjecting this fraction to affinity chromatography using, for example, Con-A Sepahrose, followed by chromatofoucusing by means of high-performance liquid chromatography for proteins ( e.g., FPLC/Mono-P ). The KBS-$\alpha$ adsorbed on DEAE-Sephadex A-50 can be purified by elution with a suitable eluent, and subjecting the eluate to affinity chromatography using, for example, Con-A Sepharose, followed by purification through chromatofocusing by means of, for example, high-performance liquid chromatography for proteins ( e.g., FPLC/Mono-P ). Alternatively, KBS-$\alpha$ can be purified by column chromatography on DEAE-Sephadex A-50 which does not adsorb

KBS-$\beta$ and KBS-$\gamma$. When treating a supernatant containing KBS-$\alpha$ directly with an anion exchanger, such as DEAE-Sephadex A-50, KBS-$\alpha$ is not adsorbed and transferred to the liquid fraction. After concentrating this liquid fraction containing KBS-$\alpha$ by using of polyethylene glycol 6000 or through ultrafiltration ( e.g., Amicon concentrator or Pellicon cassette ), followed, as required, by dialysis against a buffer solution of low salt concentration, the concentrate is again passed through a column packed with DEAE-Sephadex A-50. This time KBS-$\alpha$ is adsorbed on DEAE-Sephadex A-50. The adsorbed KBS-$\alpha$ fraction is eluted with a suitable eluent, the eluate is purified by affinity chromatography using, for example, Con-A Sepharose, and the adsorbed KBS-$\alpha$ is finally purified by chromatofocusing by means of high-performance liquid chromatography for proteins ( e.g., FPLC/Mono-P ).

KBS-$\alpha$, KBS-$\beta$ and KBS-$\gamma$ can be separately purified by the methods described above. It is also possible to obtain a purified fraction containing all of these three factors or any desired two of the three factors by suitable combination of purification techniques mentioned above. These purified fractions, of course, are included in the human endogenous cancer regulatory factors of the present invention. Both KBS and KBS-$\alpha$ are adsorbed on DEAE-Sephadex A-50, but these can be separated, for example, by employing either a different amount of the ion exchanger or a different salt concentration.

The human endogenous cancer regulatory factors prepared, recovered and purified as detailed above have relative molecular weights ( as measured by the molecular sieve method using Sephadex G-200 ) and isoelectric points (as measured by chromatofocusing by means of high-performance liquid chromatography; FPLC/Mono-P ) as given below.

| | Relative Molecular Weight | Isoelectric Point |
|---|---|---|
| KBS-$\alpha$ | 60,000 ± 10,000 | 6.5 ± 0.5 |
| KBS-$\beta$ | 60,000 ± 10,000 | 7.0 ± 0.5 |
| KBS-$\gamma$ | 62,000 ± 10,000 | 8.0 ± 0.5 |

The anti-tumor activities of KBS-$\alpha$, KBS-$\beta$ and KBS-$\gamma$ of the present invention are described below.

Cells ( $5 \times 10^5$ ) of mouse-derived, methylchoranthrene-induced sarcoma ( Meth A ), serially maintained in Balb/C mice, were transplanted into the dorsal skin of Balb/C mice ( male, 7-week old ). After seven days, the size of tumors developed was measured and 0.5ml of increasing dilutions of KBS-$\alpha$, KBS-$\beta$ and KBS-$\gamma$ ( in the range from 3,200 to 400 units ) was intravenously injected.

Tumor size measurement nine days after administration revealed that complete cure was observed for all the test groups administered with 3,200 to 1,600 units. With all the mice on which any reduction in tumor size was noticed, tumor size continued to decrease from then on, with the transplanted Meth A finally disappearing completely.

-13-

With the control group, on the other hand, the size of transplanted Meth A increased to a size more than 1.5cm, and nearly all of the mice dyed by the time 40 days later. The powerful anti-tumor activities of KBS-$\alpha$, KBS-$\beta$ and KBS-$\gamma$ of the present invention is apparent from the result of this experiment.

It was also demonstrated that KBS-$\alpha$, KBS-$\beta$ and KBS-$\gamma$ of the present invention have remarkable tumor necrosis activity when tested on nude mice transplanted with human-derived, gastric cancer cell line  MKN-45 ( serially maintained on nude mice ) according to the method of Haranaka, et . al. [ The Japanese Journal of Clinical Medicine, 40, No.8, pp. 186-193 ( 1982 )].   These factors also proved effective against Sarcoma 180, Ehrlich solid tumor, P388 solid tumor and Lewis lung carcinoma.

The biological activity ( KBS activity ) of the human endogenous cancer regulatory factors ( KBS-$\alpha$, KBS-$\beta$ and KBS-$\gamma$ ) of the present invention was evaluated by an in-vitro cytotoxicity test using normal or malignant mammalian cells according to the procedure reported by Carswell, et al. [ Proc. Nat. Acad. Sci. USA, 72, No.9, pp.3666-3670 ( 1975 )].   The test was conducted using a 96-well microplate ( Nunk Inc. of Denmark ), Eagle's minimum essential medium ( MEM medium ) containing 10% fetal calf serum, 50 $\mu$g/ml of kanamycin, 25 $\mu$g/ml of streptomycin, 50 unit/ml of gentamicin or sisomicin, and L cell (S). A suspension of $2.5 \times 10^4$ cells and diluted samples of eqaul volume were incubated on the MEM medium at 37°C for 48 hours in an atmosphere of air containing 5% carbon dioxide. 1 unit (U) is defined by a reciprocal dilution of the sample to effect 50% killing of the L cells.

The human endogenous cancer regulatory factors of the present invention, when used as medicine, are commonly administered in a form that allows full exhibition of their anti-tumor effects. Although these factors may be immediately administered, it is also possible to offer them, according to pharmaceutical practice, as a mixture with pharmaceutically acceptable diluents and/or other pharmacologically effective substances. Thus KBS-$\alpha$, KBS-$\beta$ and KBS-$\gamma$ are capable of assuming any desired form suitable for oral or parenteral administration, such as powders, granules, tablets, sugar-coated tablets, capsules, pills, suppositories, suspensions, liquids, emulsions, injections and aerosols. Typically, these factors are previously lyophilized, and administered to patients intravenously, subcutaneously or intramuscularly after being dissolved, prior to use, in physiological salines, sterile water or aseptic isotonic solution for injection. It is also possible to add, prior to lyophilization, suitable stabilizers such as mannitol and human serum albumin, as well as solubilizing agents such as glycine.

The minimum daily dose of KBS-$\alpha$, KBS-$\beta$ and KBS-$\gamma$ of
the present invention for human adults is 10,000 units.
However, the suitable dose may vary depending on patient's
sensitivty, age, sex, body weight, mode, timing and interval of administration, patient's conditions, type and properties of formulation, and other factors.   Therefore,
the dose level mentioned above should be considered as
a guide figure; a lower  dose may serve the purpose in
some cases, and a higher dose may be necessary in some .
other cases.

It is also important to note that the present invention
includes a basic technology to produce KBS-$\alpha$, KBS-$\beta$ and
KBS-$\gamma$ of the present invention by utilizing genetic engineering techniques.   Some examples will be described below.
Normal or leukemic  human monocytic cells are grown in
a tissue culuture medium by the method described above,
subjected to the first and second stimulation, and  cultured
for an additional 8 to 24 hours.   The grown cells are
separated by centrifugation, thoroughly washed with an
isotonic buffer solution, and destroyed or dissolved by
treatment with guanidium thiocyanate/mercaptoethanol or
a detergent such as Triton N101, followed by extraction
of released RNA with phenol.   The RNA extractedispassed

0161384

through an Oligo dT-cellulose column to adsorb mRNA with poly A, which are then fractionated by sucrose density gradient centrifugation. Each of the mRNA fractions thus obtained is introduced into hormonized *Xenopus laevis* oocytes with a micro-injector, and the oocytes are incubated for a predetermined period of time. The culture supernatant, or the supernatant obtained after destroyment of the oocytes followed by centrifugation, is fractionated, and fractions having high KBS activity are collected as crude mRNA of KBS-$\alpha$, KBS-$\beta$ or KBS-$\gamma$. Alternatively, it is possible to obtain mRNA of KBS-$\alpha$, KBS-$\beta$ or KBS-$\gamma$ of higher specificity by treating monocyte/macrophage leukemic cells, e.g., YKBS-7-15, in the smae manner as above, specifically extracting polysome capable of producing KBS-$\alpha$, KBS-$\beta$ or KBS-$\gamma$ by the antibody affinity technique, and passing the extracted polysome through an Oligo dT-cellulose column.

The mRNA thus obtained may be converted to cDNA by using a suitable transcriptase, incorporating the cDNA into a cloning vector such as pBR322, and introducing the vector into a host bacterium to effect transformation. One can thus produce, by the usual positive or negative screening technique, the corresponding mRNA or cDNA of high specificity. The human endogenous cancer regulatory factors of the present invention, KBS-$\alpha$, KBS-$\beta$ and

-17-

KBS-$\gamma$, can be produced from these host cells containing a cloning vector that carries DNA segment coding for the factors.

The following examples will further illustrate the present invention but are not to be considered a limitation thereupon.


Formulation  Example ( Injection )

One million units of KBS-$\alpha$, KBS-$\beta$ and/or KBS-$\gamma$ of the present invention were dissolved in 100ml of physiological saline, and the solution was filtered germfree through a membrane filter.   One-milliliter aliquots of the filtrate were charged into sterile vials and then lyophilized.


## Example 1

YK3S-4B3 —— a cloned strain of YKBS-7-15 ( monocytic leukemic cells ) which is an established cell line derived from peripheral buffy coat leukocytes of a monocytic leukemia patient —— was grown in 8 liters of RPMI 1640 medium ( Gibco Co.), which is a commonly used basic growth      medium containing 10% fetal calf serum, at 37°C for a sufficient period of time with agitation.   12-O-tetradecanoylphorbol-13-acetate ( TPA; 5 ng/ml ) was added to effect first stimulation, 1 $\mu$g/ml of endotoxin ( lipopolysaccharide derived

-18-

from E. coli 0111; B4 ) was added 36 hours later as the second stimulant, cell culture was continued for an additional 16 hours, and the culture supernatant was collected after centrifugation.

This supernatant was diluted with 20mM Tris-HCl buffer ( pH: 7.8 ) until the final salt concentration fell below 0.04M, and the diluted solution was allowed to flow batchwise through a Buchner funnel loaded with DEAE-Sephadex A-50 previously equilibrated with 20mM Tris-HCl buffer containing 0.04M sodium chloride ( pH: 7.8 ), to recover the unadsorbed fraction.

a) The fraction not adsorbed on DEAE-Sephadex A-50 was concentrated by means of a Pellicon ultrafiltration concentrator, the salt concentration was adjusted to 0.5M sodium chloride, and the resulting solution was subjected to affinity chromatography using Concanavalin A ( Con A ) Sepharose previously equilibrated with a buffer solution containing 0.5M sodium chloride ( pH: 7.8 ). The resin was thoroughly washed with the same buffer to remove unadsorbed substances and then eluted with 20mM Tris-HCl buffer solution containing α-methylmannoside and 0.5M sodium chloride ( pH: 7.8 ). The fraction adsorbed on Con-A Sephadex thus eluted was concentrated, dialyzed overnight against 25mM triethanolamine/iminodiacetic acid buffer solution ( pH: 8.1 ), and subjecting to chromatofocusing by means of high-perfor- mace liquid chromatography for proteins ( FPLC/Mono P column; Pharmcia AB ) to effect isoelectric fractionation ( linear concentration gradient method over the pH range from 8.1 to 5.0 ), giving two KBS-activity peaks in the vicinity of about pH 7.0 (KBS-ß) and about pH 8.0 (KBS-γ).

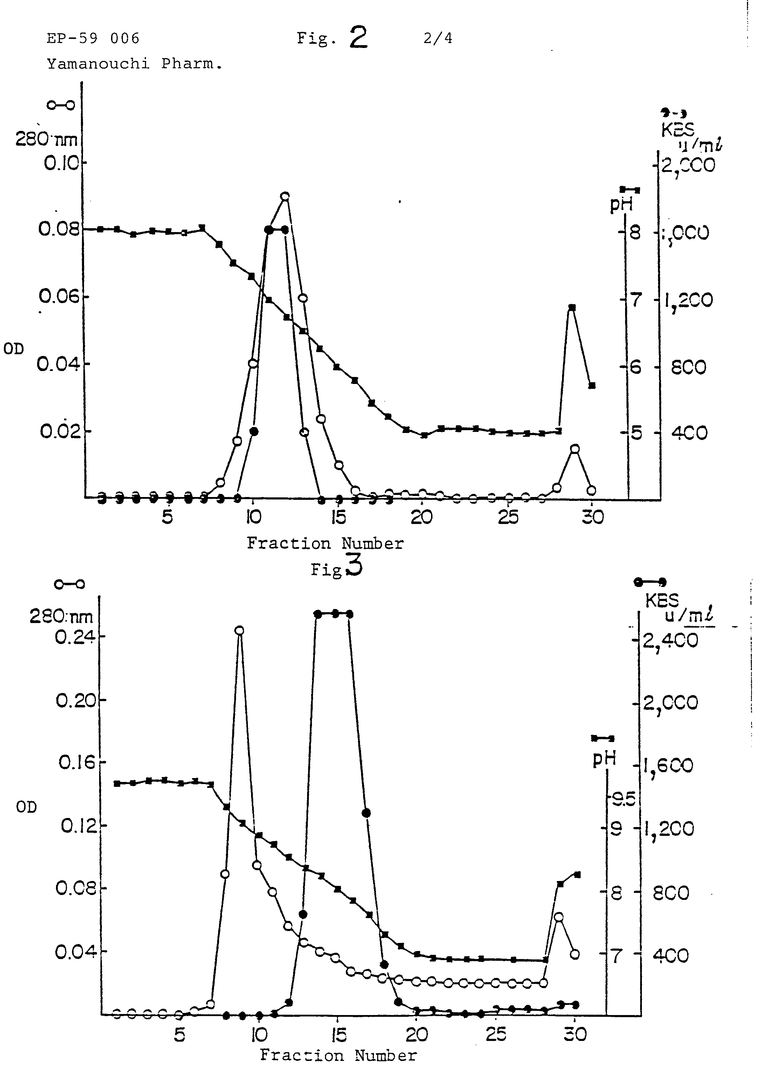

The elution profile is shown in Figure 1. Active fractions ( ⊢————⊣ KBS-ß and ⊢————⊣ KBS-γ in Fig1. 1 ) were collected. The KBS-ß fraction was dialyzed overnight against 25mM triethanolamine/iminodiacetic acid buffer solution ( pH: 8.1 ) and subjected to chromatofocusing using FPLC, Mono P column in the same manner as above, giving KBS-ß as a single sharp peak at pH of about 7.0. The elution profile is shown in Fig. 2. The isoelectric point of KBS-ß estimated was pH 7.0 ± 0.5. The KBS-γ fraction was dialyzed overnight against 25mM diethanolamine/HCl buffer solution ( pH: 9.5 ) and subjected to chromatofocusing using FPLC/Mono P column ( linear concentration gradient method over the pH range from 9.5 to 6.8 ), giving KBS-γ as a single sharp peak at pH of about 8.0. The elution profile is shown in Fig. 3. The isoelectric point of KBS-γ estimated was pH 8.0 ± 0.5. The total cytotoxicity of these fractions against L cells was 4.0 x $10^4$ units for KBS-ß and 1.0 x $10^4$ for KBS-γ.

Part of the KBS-ß and KBS-γ fractions each was passed through a Sephadex G-200 ( Pharmacia

AB ) column ( $\phi$5 x 1,400mm ). The result of chromatography showed that KBS-$\beta$ has a molecular weight of 60,000 ± 10,000 and KBS-$\gamma$ has a molecular weight of 62,000 ± 10,000. The elution profiles are shown in Figs. 4 and 5, respectively.

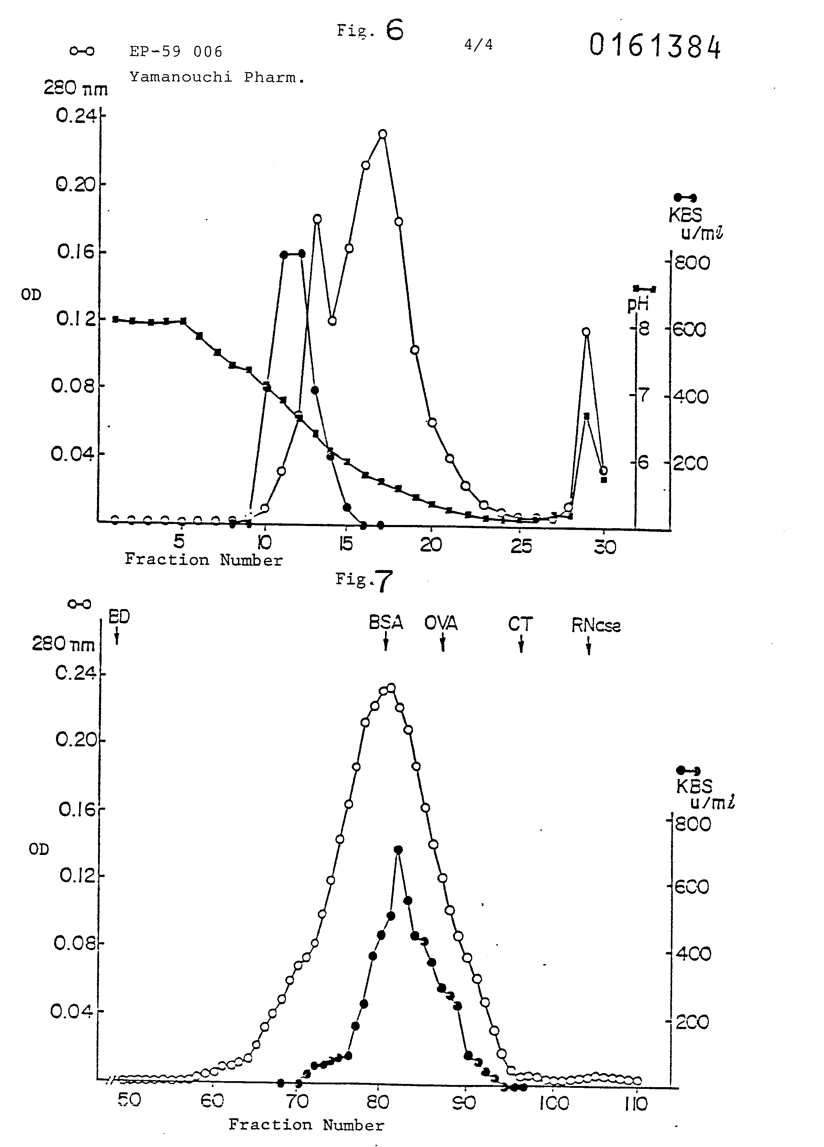

b) The fraction left adsorbed on DEAE-Sephadex A-50 was eluted with 20mM Tris-HCl buffer solution containing 0.1M sodium chloride ( pH: 7.8 ), the eluate was concentrated, and its salt concentration was adjusted to 0.5M sodium chloride. The resulting solution was subjected to affinity chromatography using a Con-A Sepharose column in the same manner as a), the adsorbed fraction was concentrated, dialyzed overnight, and then subjected to chromatofocusing using FPLC Mono P column ( linear concentration gradient method over the pH range from 8.1 to 5.0 ), giving a peak of KBS activity at pH of about 6.5 ( KBS-$\alpha$ ). The elution profile is shown in Fig. 6. The isoelectric point of KBS-$\alpha$ estimated was pH 6.5 ± 0.5. The total cytotoxic activity of this fraction against L cells was 2.5 x $10^4$ units.

Part of this KBS-$\alpha$ fraction was passed through a Sephadex G-200 ( Pharmacia AB ) column ( $\phi$5 x 1,400mm ). The result of chromatography showed that KBS-α has a relative molecular weight of 60,000 ± 10,000. The elution profile is shown in Fig. 7.

When the culture supernatant was subjected, without

-21-

being diluted, to treatment with DEDA-Sephadex A-50 and the adsorbed fraction was eluted with 20mM Tris-HCl buffer solution containing 0.04M sodium chloride, KBS which we formerly isolated could be obtained.

Table I shows the stability of KBS-$\alpha$, KBS-$\beta$ and KBS-$\gamma$ against heat and changes in pH.

Table I

| pH* | Residual Activity (U/ml) | | | Retention (%) | | |
|---|---|---|---|---|---|---|
| | KBS-$\alpha$ | KBS-$\beta$ | KBS-$\gamma$ | KBS-$\alpha$ | KBS-$\beta$ | KBS-$\gamma$ |
| Control (untreated) | 2,560 | 1,280 | 1,280 | 100 | 100 | 100 |
| 2.5 | 20 | 20 | 0 | 0.8 | 1.6 | 0 |
| 3.5 | 28 | 20 | 20 | 1.1 | 1.6 | 1.6 |
| 4.5 | 2,560 | 1,280 | 1,280 | 100 | 100 | 100 |
| 6.0 | 2,560 | 1,280 | 1,280 | 100 | 100 | 100 |
| 7.5 | 2,560 | 1,280 | 1,280 | 100 | 100 | 100 |
| 9.5 | 2,560 | 1,280 | 1,280 | 100 | 100 | 100 |
| 10.5 | 1,800 | 1,280 | 1,280 | 70.3 | 100 | 100 |
| 11.5 | 640 | 640 | 320 | 25 | 50 | 25 |
| Heat Treatment** | | | | | | |
| 56°C/30' | 2,560 | 1,280 | | 100 | 100 | |

\* Stability against pH changes was measured by the equilibrium dialysis method ( at room temperature for 17 hours against buffer solution of each pH ).

\*\* Heat stability was measured by treating each sample dissolved in buffer solution of pH 7.8 under the specified conditions.

Example 2

YK3S-7-15 ( monocytic leukemic cells),
derived from peripheral buffy coat leukocytes of
monocytic leukemia patient, were grown in 8 liters of RPMI 1640
medium ( Gibco Co. ), which is a commonly used basic
growth    medium containing 10% fetal calf serum, at 37°C
for a sufficient period of time, 5 ng/ml of 12-0-tetradecan-
oylphorbol-13-acetate ( TPA ) was added to induce first
stimulation, 1 µg/ml of endotoxin ( lipopolysaccharide
derived from E. coli; B4 ) was added as the second stimulant
36 hours later, cell culture was continued for an additional
16 hours, and the culture supernatant was collected after centrifugation.

This supernatant was diluted with 20mM Tris-HCl buffer
solution ( pH: 7.8 ) until the final salt concentration
fell below 0.04M, and the diluted solution was allowed
to flow batchwise through a Buchner funnel packed with
DEAE-Sephadex A-50 previously equilibrated with 20mM Tris-
HCl buffer containing 0.04M sodium chloride ( pH: 7.8 ),
to recover the unadsorbed fraction.
The fraction adsorbed on the DEAE-Sephadex A-50 was
eluted with 20mM Tris-HCl buffer solution containing 0.1M
sodium chloride, the eluate was concentrated, and its

-23-

salt concentration was adjusted to 0.5M sodium chloride. The resulting solution was then subjected to affinity chromatography using a Con-A Sepharose column in the same manner as a) in Example 1, and the adsorbed fraction was concentrated, dialyzed overnight, and subjected to chromatofocusing using FPLC/Mono P column to effect isoelectric fractionation ( linear concentration gradient method over the pH range from 8.1 to 5.0 ), giving a peak of KBS activity at pH of about 6.5 ( KBS-$\chi$ ). Dialysis of this active fraction overnight afforded purified KBS-$\chi$.

When the culture supernatant was subjected, without being diluted, to treatment with DEAE-Sephadex A-50 and the adsorbed fraction was eluted with 20mM Tris-HCl buffer solution containing 0.04M sodium chloride, the KBS we formerly isolated could be obtained.

## Example 3

YKBS-4B3, which is a cloned cell lineof YKBS-7-15, was grown in 8 liters of serum-free medium HB101[TM] ( Hana Biologics Inc. ) at 37°C for a sufficient period of time with agitation. TPA ( 5 ng/ml ) was added to induce first stimulation, 1 ug/ml of endotoxin ( lipopolysaccharide derived from E. coli 0111; B4 ) was added 36 hours later as the second stimulant, cell culture was continued for an additional 16 hours, and the culture supernatant was collected

-24-

after centrifugation.

This supernatant was concentrated by means of a Pellicon ultrafiltration concentrator at a factor of about 20, and saturated aqueous ammonium sulfate solution was slowly added to the concentrate until the final concentration of ammonium sulfate reached 50% ( v/v ). The precipitate thus formed was allowed to stand at 4°C to ensure complete aging, collected by centrifugation, dissolved in a small volume of 20mM Tris-HCl buffer solution containing 0.04M sodium chloride ( pH: 7.8 ), and dialyzed sufficiently against the same buffer. The dialyzed solution was allowed to flow through a Buchner funnel loaded with DEAE-Sephadex A-50 previously equilibrated with the same buffer, and the adsorbed substance was eluted with Tris-HCl buffer containing 0.08M sodium chloride ( pH: 7.8 ). The eluate was concentrated by means of a Pellicon ultrafiltration concentrator to adjust the salt concentration to 0.5M sodium chloride, and the resulting solution was subjected to affinity chromatography using Concanavalin A ( Con A ) Sepharose previously equilibrated with Tris-HCl buffer solution containing 0.5M sodium chloride ( pH: 7.8 ). The resin was thoroughly washed with the same buffer to remove unadsorbed substances and then eluted with 20mM Tris-HCl buffer solution containing ⍺-methylmannoside and 0.5M sodium chloride ( pH: 7.8 ), giving a fraction containing KBS-⍺, KBS-β

and KBS-$\gamma$. This fraction was subjected to chromatofocusing by means of high-performance liquid chromatography for proteins ( FPLC/Mono P column; Pharmcia AB ) to effect isoelectric fractionation ( linear concentration gradient method ), affording three peaks of KBS-activity at pH of about 8.0 ( KBS-$\gamma$ ), about 7.0 ( KBS-$\beta$ ) and about 6.5 ( KBS-$\alpha$ ).

## Example 4

YKBS-4B3, which is a cloned cell line of YKBS-7-15, was grown in 8 liters of serum-free medium HB101$^{TM}$ ( Hana Bio-logics Inc. ) at 37°C for a sufficient period of time with agitation. TPA ( 5 ng/ml ) was added to induce first stimulation, 1 $\mu$g/ml of endotoxin ( lipopolysaccharide derived from E. coli 0111; B4 ) was added 36 hours later as the second stimulant, cell culture was continued for an additional 16 hours, and the culture supernatant was collected after centrifugation.

This supernatant was concentrated by means of a Pellicon ultrafiltration concentrator at a factor of about 20, and saturated aqueous ammonium sulfate solution was slowly added to the concentrate until the final concentration of ammonium sulfate reached 50% ( v/v ). The precipitate thus formed was allowed to stand at 4°C to ensure complete aging, collected by centrifugation, dissolved in a small volume of 20mM Tris-HCl buffer solution containing 0.04M sodium chloride ( pH: 7.8 ), and dialyzed sufficiently against the same buffer. The dialyzed solution was allowed to flow through a Buchner funnel loaded with DEAE-Sephadex A-50 previously equilibrated with the same buffer, and the adsorbed substance was eluted with Tris-HCl buffer containing 0.08M sodium chloride ( pH: 7.8 ). The eluate was concentrated by means of a Pellicon ultrafiltration concentrator to adjust the salt concentration to 0.5M sodium chloride, and the resulting solution was subjected to affinity chromatography using Concanavalin A ( Con A ) Sepharose previously equilibrated with Tris-HCl buffer solution containing 0.5M sodium chloride ( pH: 7.8 ). The resin was thoroughly washed with the same buffer to collect unadsorbed fraction, which was concentrated and dialyzed overnight against 25mM triethanolamine/iminodiacetic acid buffer solution, affording a fraction containing KBS-$\beta$ and KBS-$\gamma$ This fraction was subjected to chromatofocusing by means of high-performance liquid chromatography for proteins ( FPLC /Mono P column; Pharmcia AB ) to effect isoelectric fractionation ( linear concentration gradient method ), giving two peaks of KBS-activity at pH of about 7.0 ( KBS-$\beta$) and about 8.0 ( KBS-$\gamma$ ).

Patentanwälte
Wuesthoff - v. Pechmann
Behrens - Goetz
Schweigerstraße 2
8000 München 90

WHAT IS CLAIMED IS:

1. A human endogenous cancer regulatory factor having a molecular weight not smaller than 50,000 and less than 72,000 and having an isoelectric point in the pH range from 6.0 to 8.5.

2. A human endogenous cancer regulatory factor according to Claim 1 wherein said cancer regulatory factor is KBS-α having a molecular weight not smaller than 50,000 and less than 70,000 and having an isoelectric point in the pH range of 6.5 ± 0.5.

3. A human endogenous cancer regulatory factor according to Claim 1 wherein said cancer regulatory factor is KBS-β having a molecular weight not smaller than 50,000 and less than 70,000 and having an isoelectric point in the pH range of 7.0 ± 0.5.

4. A human endogenous cancer regulatory factor according to Claim 1 wherein said cancer regulatory factor is KBS-γ having a molecular weight not smaller than 50,000 and less than 72,000 and having an isoelectric point in the pH range of 8.0 ± 0.5.

5. A process for producing a human endogenous cancer regulatory factor having a molecular weight not smaller than 50,000 and less than 70,000 and having an isoelectric point in the pH range from 6.0 to 8.5 by growing human monocytic cells capable of producing said factor or a cloned

-28-

cell (line) therefrom in a tissue culture medium and recovering the thus produced human endogenous cancer regulatory factor from said tissue culture medium.

6. The process according to Claim 5 wherein human, normal or monocytic leukemic cells, or a cloned cell line therefrom, is grown in a tissue culture medium in the presence of a first stimulant and then in the presence of a second stimulant, followed by recovery, from said tissue culture medium, of the thus produced human endogenous cancer regulatory factor having a molecular weight not smaller than 50,000 and less than 72,000 and having an isoelectric point in the pH range from 6.0 to 8.5.

7. The process according to Claim 5 wherein said leukemic cells are YKBS-7-15.

8. The process according to Claim 5 wherein said cloned cell line is YKBS-4B3.

9. The process according to Claim 5 wherein said tissue culture medium is a growth medium containing fetal calf serum.

10. The process according to Claim 6 wherein said first stimulant is TPA.

11. The process according to Claim 6 wherein said second stimulant is a lipopolysaccharide.

12. A pharmaceutical composition containing KBS-𝛼, KBS-𝛽 or KBS-𝛾 alone or in combination.

13. The pharmaceutical composition according to Claim 12 wherein said composition is a parenteral injection.

EP-59 006
Yamanouchi Pharm.

Patentanwälte
Wuesthoff - v. Pechmann
Behrens - Goetz 0161384
Schweigerstraße 2
8000 München 90

Claims for Austria:

1. A process for producing a human endogenous cancer regulatory factor having a molecular weight not smaller than 50,000 and less than 72,000 and having an isoelectric point in the pH range from 6.0 to 8.5 by growing human monocytic cells capable of producing said factor or a cloned cell line therefrom in a tissue culture medium and recovering the thus produced human endogenous cancer regulatory factor from said tissue culture medium.

2. The process according to Claim 1 wherein said cancer regulatory factor is KBS-$\alpha$ having a molecular weight not smaller than 50,000 and less than 70,000 and having an isoelectric point in the pH range of 6.5 ± 0.5.

3. The process according to Claim 1 wherein said cancer regulatory factor is KBS-$\beta$ having a molecular weight not smaller than 50,000 and less than 70,000 and having an isoelectric point in the pH range of 7.0 ± 0.5.

4. The process according to Claim 1 wherein said cancer regulatory factor is KBS-$\gamma$ having a molecular weight not smaller than 50,000 and less than 72,000 and having an isoelectric point in the pH range of 8.0 ± 0.5.

- 2 -

0161384

5. The process according to Claim 1 wherein human, normal or monocytic leukemic cells, or a cloned cell line therefrom, is grown in a tissue culture medium in the presence of a first stimulant and then in the presence of a second stimulant, followed by recovery, from said tissue culture medium, of the thus produced human endogenous cancer regulatory factor having a molecular weight not smaller than 50,000 and less than 72,000 and having an isoelectric point in the pH range from 6.0 to 8.5.

6. The process according to Claim 5 wherein said leukemic cells are YKBS-7-15.

7. The process according to Claim 5 wherein said cloned cell line is YKBS-4B3.

8. The process according to Claim 1 wherein said tissue culture medium is a growth medium containing fetal calf serum.

9. The process according to Claim 5 wherein said first stimulant is TPA.

10. The process according to Claim 5 wherein said second stimulant is a lipopolysaccharide.

11. A pharmaceutical composition containing KBS-$\alpha$, KBS-$\beta$ or KBS-$\gamma$ alone or in combination.

12. The pharmaceutical composition according to Claim 11 wherein said composition is a parenteral injection.

0161384

13. A human endogenous cancer regulatory factor having a molecular weight not smaller than 50,000 and less than 72,000 and having an isoelectric point in the pH range from 6.0 to 8.5.

Fig. 1

Fig. 2

Fig. 3

EP-59 006
Yamanouchi Pharm.

Fig. 4  3/4

0161384

Fig. 5

Fig. 6

4/4

0161384

o—o EP-59 006
Yamanouchi Pharm.

Fig. 7